# EUROPEAN PATENT APPLICATION

(11) **EP 4 375 359 A1**
(43) Date of publication of application: **29.05.2024**
(21) Application number: 22845979.8
(22) Date of filing: 19.07.2022
(51) Int. Cl.: C12M 1/00, C12M 3/00, C12N 5/077, C12N 5/0775

(54) **CULTURE SYSTEM**

(30) Priority: 20.07.2021 JP 2021119592
(71) Applicant: SAGA UNIVERSITY, Saga-shi, Saga 840-8502 (JP)
(72) Inventor: NAKAYAMA, Koichi, Saga-shi, Saga 840-8502 (JP); NAKAMURA, Anna Maria, Saga-shi, Saga 840-8502 (JP)
(74) Representative: J A Kemp LLP
(86) International application number: PCT/JP2022/028554
(87) International publication number: WO 2023/003046

(57) **Abstract**

A culture system that comprises: a support for stacking cell aggregates, said support being provided with needle-shaped parts that are arranged on a substrate like a kenzan (flower frog needles), in which the outermost needle-shaped parts on the substrate are arranged so that the distance from the needle-shaped parts to the outer edge of the substrate is shorter than the distance between adjacent needle-shaped parts; a culture tank for housing a culture unit in which cell aggregates are stacked on the support; a liquid culture medium container; and a plurality of liquid culture medium supply pipes that supply a liquid culture medium from the liquid culture medium container to the culture unit, wherein each of the plurality of liquid culture medium supply pipes is positioned between the needle-shaped part.

## Description

### TECHNICAL FIELD

The present invention relates to a culture system using a support for stacking cell aggregates, the support having needle-shaped parts arranged on a substrate like a Kenzan (spiky flower frog), and to a method for producing a large-size three-dimensional cellular structure.

### BACKGROUND ART

A method for producing a cellular construct consisting solely of cells is known, in which method cell masses are arranged in a predetermined three-dimensional space using a support for arranging the cell masses in the predetermined space and the cell masses are allowed to fuse with each other (Japanese Patent No. 4517125), and automated devices have been developed for this method (Non-patent literature 1: D. Murata, K. Arai, and K. Nakayama, "Scaffold-Free Bio-3D Printing Using Spheroids as `Bio-Inks' for Tissue (Re-)Construction and Drug Response Tests," Adv. Healthc. Mater., vol. 9, no. 15, p. 1901831, Aug. 2020; Non-patent literature 2: K. Nakayama, Kenzan Method for Scaffold-Free Biofabrication. Cham: Springer International Publishing, 2021.)

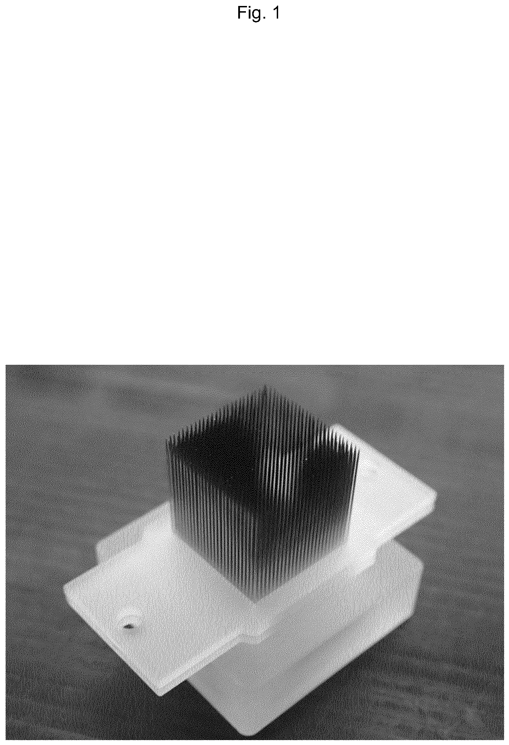

The above device can be used as a bio-3D printer to construct cellular structures of various shapes. In the above device, a support having needle-shaped parts arranged within 1 cm square is used (Figure 1). Accordingly, the maximum size (area of the plane) of stacking (printing) is 1 cm².

Therefore, in order to print a cellular structure of a larger size, it is necessary to increase the area for placing the needle-shaped parts and to increase the number of needle-shaped parts.

### CITATION LIST

### [Patent literature]

[Patent literature 1] Japanese Patent No. 4517125

### [Non-patent literature]

[Non-patent literature 1] D. Murata, K. Arai, and K. Nakayama, "Scaffold-Free Bio-3D Printing Using Spheroids as `Bio-Inks' for Tissue (Re-)Construction and Drug Response Tests," Adv. Healthc. Mater., vol. 9, no. 15, p. 1901831, Aug. 2020.
[Non-patent literature 2] K. Nakayama, Kenzan Method for Scaffold-Free Biofabrication. Cham: Springer International Publishing, 2021

### SUMMARY OF INVENTION

### Technical Problems

Printing a large-size cellular structure requires manipulations such as increasing the size of the support, but this requires modifications to the bioprinter itself, which is burdensome and costly.

For this reason, there has been a need for a means for easily increasing the size of a cellular structure.

### Solution to Problem

As a result of a diligent study to solve the above problem, the present inventor succeeded in constructing a large-size cellular structure by modularizing (componentizing) Kenzan-shaped supports, in other words, dividing the 3D data of the target cellular structure, stacking cell aggregates on the supports which have been modularized for the respective divided data, and culturing these supports, thereby completing the present invention.

Thus, the present invention is as follows.
(1) A support for stacking cell masses, the support comprising needle-shaped parts arranged like a Kenzan (spiky flower frog) on a substrate, wherein the outermost needle-shaped part on the substrate is arranged such that the distance from said needle-shaped part to the outer edge of the substrate is shorter than the distance between adjacent needle-shaped parts.
(2) The support according to (1), wherein the distance from the outermost needle-shaped part to the outer edge is half the distance between adjacent needle-shaped parts.
(3) The support according to either one of (1) and (2), wherein the substrate comprises a parallelogram plane.
(4) The support according to (3), wherein the parallelogram plane has a side that is 10 mm in length.
(5) A culture system for culturing cell masses, comprising:
   a culture tank for housing a culture unit comprising cell masses stacked on a plurality of supports according to any one of (1) through (4);
   a culture solution container; and
   a plurality of culture solution supply pipes for supplying a culture solution from the culture solution container to the culture unit,
   wherein each of the plurality of culture solution supply pipes is placed between the needle-shaped parts.
(6) The culture system according to (5), wherein the culture unit comprises the supports which are arranged adjacent to each other.
(7) The culture system according to either one of (5) and (6), wherein each of the plurality of culture solution supply pipes is arranged along the top surface of the substrate.
(8) The culture system according to any one of (5) through (7), wherein each of the plurality of culture solution supply pipes is arranged below the cell masses.
(9) The culture system according to any one of (5) through (8), wherein each of the plurality of culture solution supply pipes is provided with an opening through which the culture solution can flow out.
(10) The culture system according to (9), wherein the opening is formed to allow the culture solution to flow out toward the cell masses.
(11) The culture system according to any one of (5) through (10), wherein each of the plurality of culture solution supply pipes extends over the substrate parallel to at least one side of the substrate.
(12) The culture system according to any one of (5) through (11), wherein the plurality of culture solution supply pipes are arranged parallel to each other.
(13) The culture system according to any one of (5) through (12), wherein the culture tank comprises a frame or a hollowed part for housing the culture unit.
(14) A method for producing a large-size three-dimensional cellular structure which has a length of at least 10 mm in any one direction, the method comprising placing a culture unit, in which cell masses are stacked on the plurality of supports according to any one of (1) through (4), in the culture system according to any one of (5) through (13); and conducting culture.
(15) The method according to (14), wherein stacking of the cell masses comprises designing shape data of tissue to be produced by dividing the shape data into a plurality of parts and stacking the cell masses on the respective supports based on the divided design data.
(16) The method according to either one of (14) and (15), wherein the culture unit comprises the stacked cell masses that are positioned to form the original shape of the three-dimensional cellular structure to be produced.
(17) A large-size three-dimensional cellular structure produced by the method according to any one of (14) through (16) and having a length of at least 10 mm in any one direction.

### Advantageous Effects of Invention

The present invention has made it possible to produce a three-dimensional structure with a length of 10 mm or more in a predetermined direction.

### BRIEF DESCRIPTION OF DRAWINGS

[Figure 1] A view showing a conventionally used support for a bio-3D printer.
[Figure 2] A side cross-sectional view of a support for stacking cell masses.
[Figure 3] A top view of a culture system according to an embodiment of the present invention.
[Figure 4] An enlarged view of a culture solution supply pipe shown in Figure 3.
[Figure 5] A side cross-sectional view showing a state where cell masses are arranged on the support for stacking cell masses.
[Figure 6] A top view showing a state where tissue is cultured using the culture units.
[Figure 7] Views showing removal of a three-dimensional structure from the culture units.
[Figure 8] Views showing removal of a three-dimensional structure from the culture units.
[Figure 9] A picture showing a culture system of the present invention.
[Figure 10] Views showing that shape data of an ear was designed by dividing it into six parts.
[Figure 11] Pictures showing the result of stacking cell masses to form a three-dimensional structure in a shape of an ear, based on the design shown in Figure 10.
[Figure 12] Views showing that shape data of a cartilage was designed by dividing it into two parts.
[Figure 13] Pictures showing the result of stacking cell masses to form a three-dimensional structure in the shape of a cartilage, based on the design shown in Figure 12.

### DESCRIPTION OF EMBODIMENTS

The present invention relates to a support for stacking cell aggregates (also referred to as cell masses or spheroids), the support comprising needle-shaped parts arranged on a substrate like a Kenzan, wherein the outermost needle-shaped part on the substrate is arranged such that the distance from said needle-shaped part to the outer edge of the substrate is shorter than the distance between adjacent needle-shaped parts.

Conventionally, a support having needle-shaped parts arranged within a 1 cm square has been used. Since, however, the substrate on which the support is formed had marginal areas (Figure 1), it was not possible to provide the needle-shaped parts adjacent to each other on the marginal areas.

The present invention provides a modularized support in which the above-mentioned marginal areas are removed to eliminate the excess edges (i.e., to make it edgeless) so that the supports can be placed side by side.

The present invention also relates to a culture system for culturing cell masses using the above support.

### 1. Support for stacking cell masses

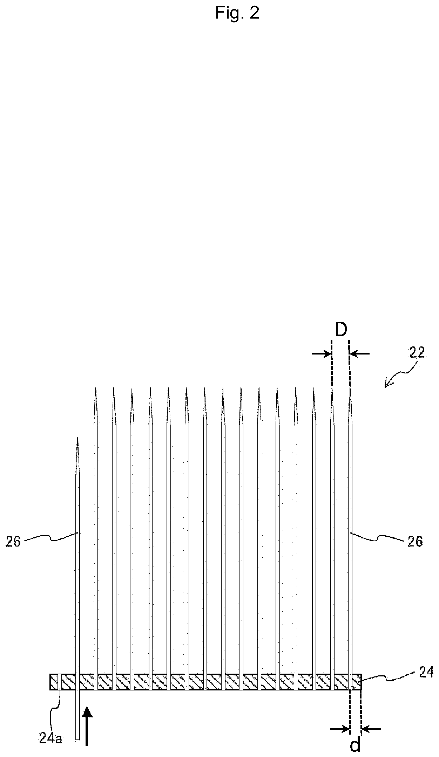

A side cross-sectional view of a support for stacking cell aggregates (cell masses) of the present invention is shown in Figure 2.

As shown in Figure 2, a support 22 for stacking cell masses (also simply referred to as a "support" or "support 22") comprises a plate-like substrate 24 having a plurality of through holes 24a and a plurality of needle-shaped parts 26 that are attached to the substrate 24 through the plurality of through holes 24a. The needle-shaped part 26 has a sharp tip and a bottom end with a diameter slightly larger than the diameter of the through hole 24a. As shown in Figure 2, when the tip of the needle-shaped part 26 is inserted into the through hole 24a from below the substrate 24 and moved upward, the bottom end of the needle-shaped part 26 fits into the through hole 24a. This secures the needle-shaped part 26 to the substrate 24. The plane of the substrate 24 may preferably have a parallelogram shape, more preferably a square or rectangular shape. The length of one side of the parallelogram (square or rectangle) may preferably be 0.5 cm-3.0 cm, more preferably 0.8 cm-1.2 cm, and still more preferably about 1 cm (10 mm).

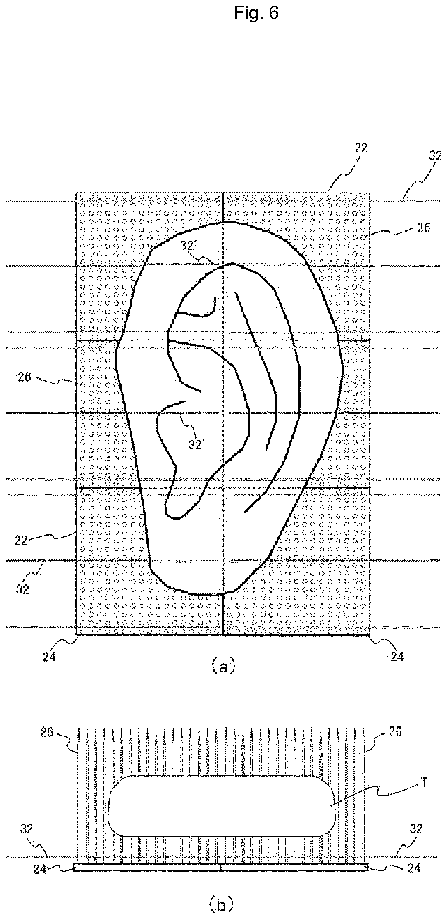

Moreover, in the support 22, the distance d from the outermost needle-shaped part 26 among the needle-shaped parts 26 attached to the substrate 24 to the outer edge of the substrate 24 is designed to be shorter than the space D between adjacent needle-shaped parts (D > d). Preferably, the distance d is half of the space D between adjacent needle-shaped parts. By eliminating the marginal area of the substrate (i.e., by making it edgeless), for example, when two supports are placed side by side, the distance between the outermost needle-shaped part on one support and the outermost needle-shaped part on the other support on the adjacent area is approximately equal to the space D (Figure 6b). When the distance d is half of the space D between the needle-shaped parts, the distance from the outermost needle-shaped part of one support to the outermost needle-shaped part of the other support on the adjacent area is 2d = D. This facilitates the adhesion of the neighboring cell aggregates on the adjacent supports.

Examples of the material used for the needle-shaped parts 26 include, but are not limited to, stainless steel, polypropylene, and nylon.

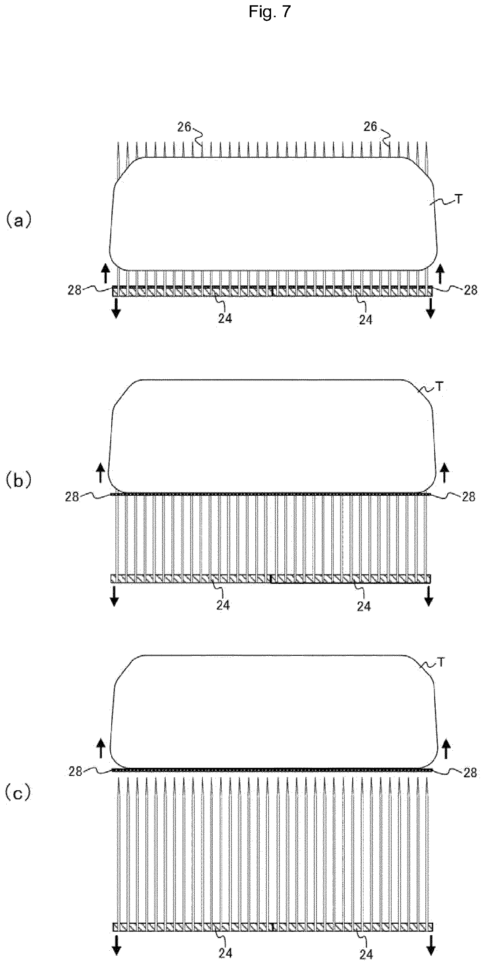

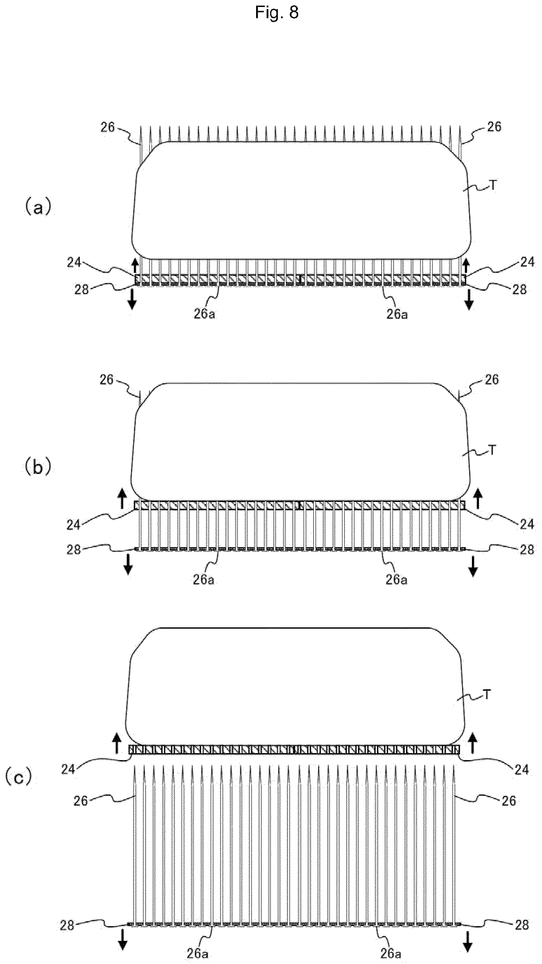

In addition, the substrate 24 and a sheet 28 (details of the sheet 28 will be described below (Figures 7 and 8)) are preferably coated with a non-cell adhesive substance such as fluorine (e.g., made of polydimethylsiloxane). However, other than those treated with fluorine, those treated with a polyhydroxyethyl methacrylate polymer can also be used. Furthermore, those made of an acrylic resin, an ABS resin, a polyester-based resin, a polycarbonate resin, polypropylene, polyethylene, polyacetal, polyether ether ketone, nylon, etc. can also be used.

### 2. Culture system

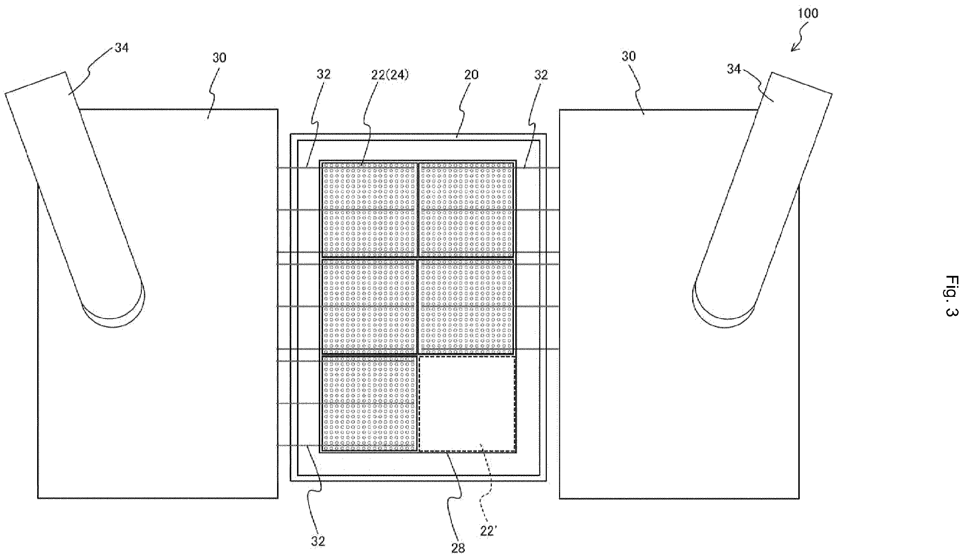

A culture system 100 according to the present embodiment is shown in Figure 3. The culture system 100 comprises a culture tank 20 for housing a plurality of supports 22, at least one culture solution container 30 for storing a culture solution, and culture solution supply pipes 32 for supplying the culture solution from the culture solution container 30 to the culture tank 20.

The culture tank 20 is provided with a frame or a hollowed part 28 for receiving the plurality of supports 22 at the bottom such that the plurality of supports 22 are positioned adjacent to each other. The frame or hollowed part 28 may be provided for each of the supports 22. The hollowed part 28 has a parallelogram shape to match the plane of the support 22, but it can also be a recess into which a projection (not shown) provided on the bottom of the support 22 can be fitted.

The substrates 24 of the adjacent supports 22 may preferably be arranged such that their sides make contact with each other.

In Figure 3, five supports 22 are arranged next to each other. Although there is no support 22 in the area 22' shown by the dashed line, an additional support 22 can be placed to have a total of six supports. The number of supports 22 to be placed can be suitably selected according to the size of a three-dimensional structure of interest.

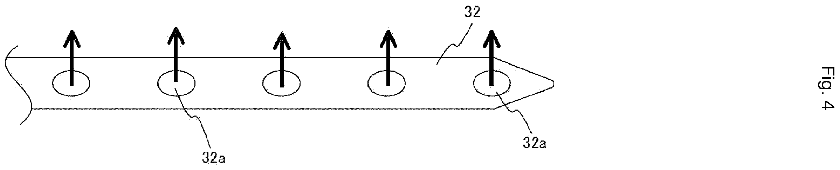

As shown in Figure 4, the culture solution supply pipe 32 has openings 32a arranged at certain intervals. The culture solution pumped from the culture solution container 30 flows out through the openings 32a. The shape of the opening 32a may preferably be circular or oval. Note that the number of openings 32a is not limited and may be one or more. Alternatively, a syringe needle may be used as the culture solution supply pipe 32 so that the tip of the needle forms the opening 32a.

As shown in Figure 3, each of the plurality of culture solution supply pipes 32 extends above the substrate 24 parallel to at least one side of the parallelogram plane of the substrate 24. The plurality of culture solution supply pipes 32 are arranged parallel to each other.

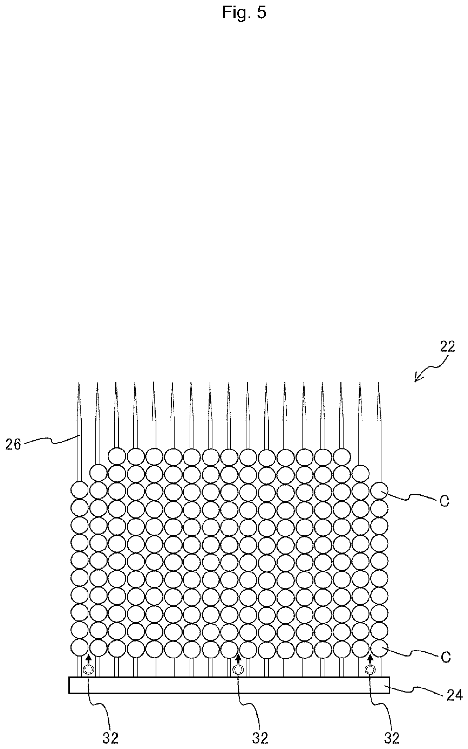

Figure 5 shows a state where a plurality of cell masses C are arranged by being penetrated by the respective needle-shaped parts 26 of the support 22. In this state, the culture solution supply pipes 32 are placed between the bottommost cell masses C and the top surface of the substrate 24, and the culture solution and other nutrient solution are supplied upward from the openings 32a of the culture solution supply pipes 32. Examples of the nutrient solution include serum and growth factors (platelet-derived growth factor (PDGF), transforming growth factor (TGF), bone morphogenetic protein (BMP), etc.), where the nutrient solution may be contained in the culture solution. Herein, a "culture solution" comprises a culture solution containing the above nutrient solution. Each of the plurality of culture solution supply pipes 32 is arranged (preferably parallel to each other) along the top surface of the substrate 24. Each of the plurality of the culture solution supply pipes 32 is arranged below the cell masses C.

The type of cells used in the present invention is not particularly limited and any cells that form a cell mass can be used. Examples of the cells that form cell masses include undifferentiated cells such as stem cells (ES cells, iPS cells, umbilical cord blood-derived cells, undifferentiated mesenchymal stem cells, adult mesenchymal stem cells, etc.) and differentiated cells thereof. Examples of tissue from which these cells are derived include articular cartilage, bone, adipose tissue, ligament, tendon, tooth, auricle, nose, liver, pancreas, blood vessel, nerve, and heart, among which cartilage or the like is preferred. The cell mass does not necessarily have to be formed as an aggregate of a single type of cells, but may be formed from multiple types of cells as long as the cell mass is formed.

In addition, the culture period for forming the cell masses is generally 3-21 days under general culture conditions (e.g., 37°C, 5% CO₂ atmosphere).

### 3. Production of three-dimensional structure

Figure 6 shows views in which shape data of a three-dimensional structure of interest (tissue T) is designed by dividing it into a plurality of parts and cell masses are stacked on the respective supports based on the divided design data, where the shape of tissue T is designed to have a shape of a human ear.

In Figure 6, the shape of the ear is broken down into six parts, and the cell masses are stacked to form each part. When the supports on which the cell masses are stacked are referred to as "culture units," Figure 6a shows six culture units arranged adjacent to each other to form the original ear shape. By culturing in the state shown in Figure 6, the neighboring cell masses on the adjacent culture units unite with each other to form a shape of a single tissue as a whole. Figure 6b is a side cross-sectional view of Figure 6a. The cell masses stacked on the left support and the cell masses stacked on the right support are fused together, thereby forming a single tissue T as a whole.

In the state shown in Figure 6, the plurality of culture solution supply pipes 32 can supply a culture solution to the tissue T (the plurality of cell masses) from the dashed-line parts 32' located beneath the tissue T consisting of the cell masses. The larger the tissue T, the more difficult it is for the culture solution and other nutrients to reach the interior of the tissue T. To avoid this, the culture system of the present embodiment is capable of supplying a sufficient amount of culture solution from the culture solution supply pipes 32 even to areas where the culture units 22 abut each other, while changing the arrangement of the culture units 22 to match the shape of the tissue T.

Once the culture is complete, the formed tissue T is taken out and isolated from the culture units.

In Figure 7, a sheet 28 is laid on top of the substrate 24 in advance (Figure 7a) so that the sheet can be lifted at the end of culture to withdraw the tissue T from the needle-shaped parts 26 (Figures 7b and 7c).

Alternatively, as shown in Figure 8a, a sheet 28 can be placed beneath the substrate 24 (i.e., the substrate 24 can be placed on the sheet 28) with the needle-shaped parts 26 penetrating therethrough to form a support 22, so that by withdrawing the sheet 28 from the substrate 24 after the culture is complete, the needle-shaped parts 26 can also be withdrawn together, thereby separating the tissue T from the culture units (Figures 8b and 8c).

The tissue T separated as described above is a large-size cellular structure with a length of at least 10 mm in any one direction.

### EXAMPLES

Hereinafter, the present invention will be described further in detail by way of examples. The scope of the invention, however, should not be limited to these examples.

### [Example 1]

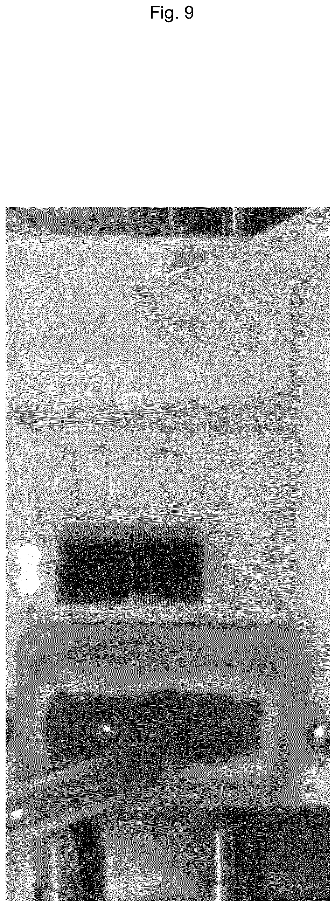

Figure 9 is a picture showing an embodiment of the culture system shown in Figure 3. Two supports were arranged in the center of the system, and culture solution supply pipes were routed from both sides.

### [Example 2]

A total of 676 (26 x 26) 0.1 mm diameter needles were arranged at 0.4 mm intervals on a square substrate 10 mm long on each side. The resultant served as a support unit (module). The distance from the outermost needle to the edge of the substrate was 0.2 mm.

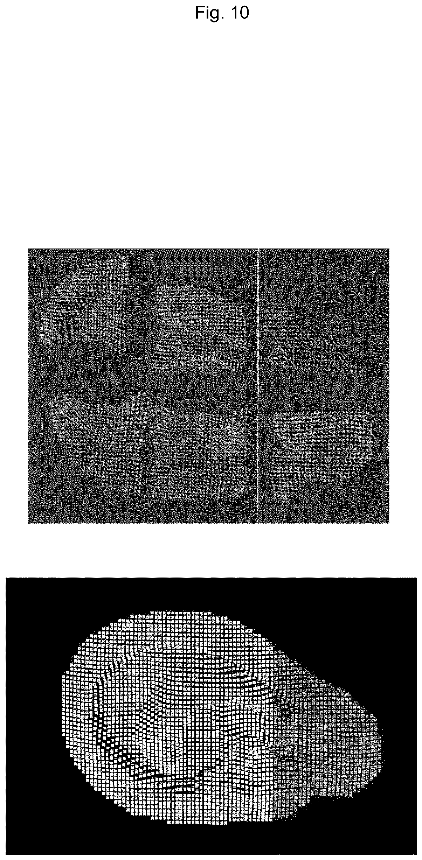

After downloading 3D data of an ear, the data was processed by dividing the shape data of the ear into six parts to stack cell masses on each part. The view on the left in Figure 10 shows the data of the ear displayed on an array of six parallelogram supports whose substrates were 1 cm square (2 cm x 3 cm culture unit as a whole). The view on the right in Figure 10 shows the data of the six ear parts.

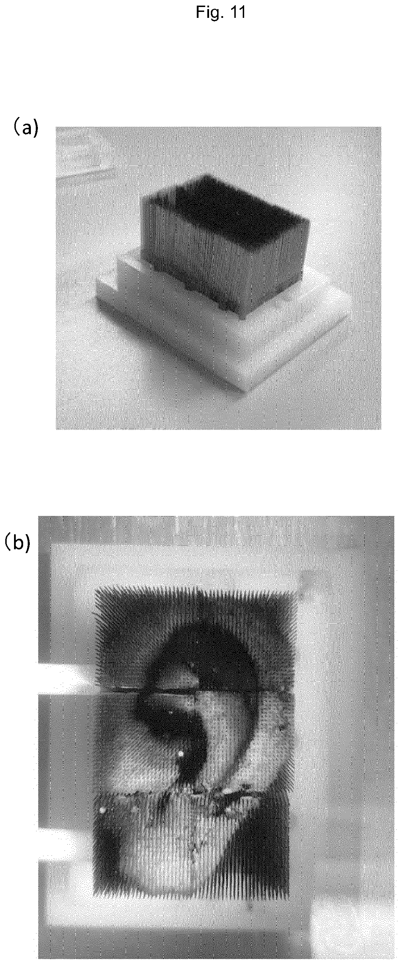

Fibroblasts were aggregated to form cell masses and the cell masses were stacked on the respective supports based on the above data. A view of the array of six supports before stacking is shown in Figure 11a. A view of the supports after stacking, which supports were arranged so that the parts were in their original arrangement, is shown in Figure 11b. As shown in Figure 11b, a three-dimensional structure formed solely from the cell masses was formed in an area 2 cm wide and 3 cm long, while maintaining the shape of the ear.

### [Example 3]

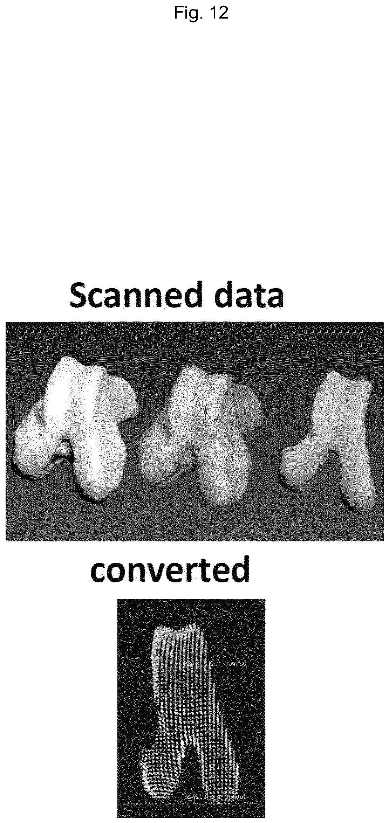

A porcine cartilage was 3D scanned and 3D data was created in the same manner as in Example 2 (upper view in Figure 12). This was divided into two parts to create data for stacking cell masses (lower view in Figure 12).

Based on this data, cell masses were stacked on each support.

The cell masses were prepared by culturing mesenchymal stem cells (iNCMSCs), which were derived from human iPS cells via neural crest, in a chondrocyte induction medium in a Prime Surface (registered trademark) 96-well plate. The culture unit having the cell masses stacked thereon was placed in the system of the present invention and cultured at 37°C using a chondrocyte induction medium.

The composition of the chondrocyte induction medium was as follows.

Basal medium (Chondrogenic Differentiation Basal Medium (PT-392, LONZA))

Serum (Chondrogenic SingleQuots (PT-4121, LONZA))

Growth factors (Platelet-Derived Growth Factor-BB (PDGF-BB), Transforming Growth Factor-β3 (TGF-03), Bone Morphogenetic Protein 4 (BMP-4))

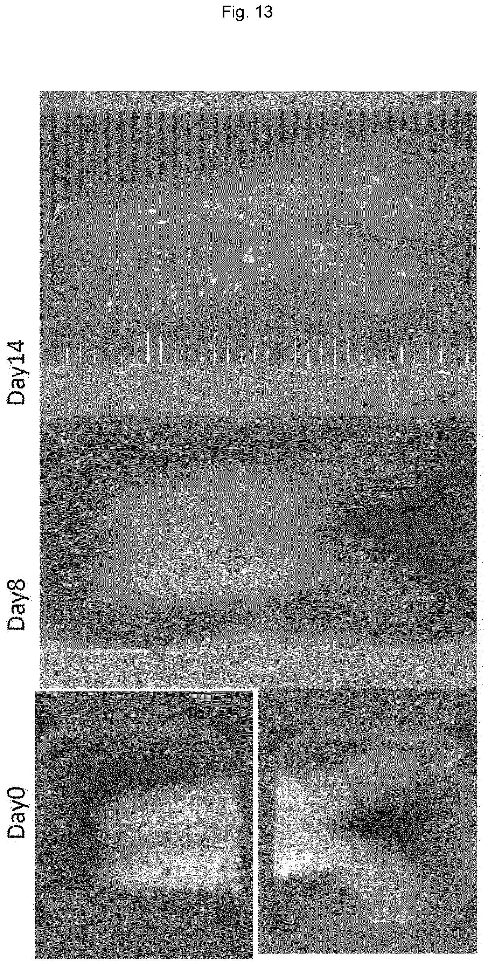

The view on the left in Figure 13 shows the cell masses on the first day of stacking (Day 0) and the view in the center in Figure 13 shows the cell masses on the 8th day of culture (Day 8). By Day 8, the cell masses were found to have fused together to form cartilage tissue with a vertical length of approximately 2 cm. The view on the right in Figure 13 shows the state on the 14th day of culture (Day 14), where the cell masses formed cartilage tissue as a whole.

### REFERENCE SIGNS LIST

- 100: Culture system
- 20: Culture tank
- 22: Support
- 24: Substrate
- 24a: Through hole
- 26: Needle-shaped part
- 28: Frame or hollowed part
- 30: Culture solution container
- 32: Culture solution supply pipe
- 32a: Opening
- C: Cell mass
- T: Tissue

## Claims

1. A support for stacking cell masses, the support comprising needle-shaped parts arranged like a Kenzan (spiky flower frog) on a substrate, wherein the outermost needle-shaped part on the substrate is arranged such that the distance from said needle-shaped part to the outer edge of the substrate is shorter than the distance between adjacent needle-shaped parts.

2. The support according to claim 1, wherein the distance from the outermost needle-shaped part to the outer edge is half the distance between adjacent needle-shaped parts.

3. The support according to either one of claims 1 and 2, wherein the substrate comprises a parallelogram plane.

4. The support according to claim 3, wherein the parallelogram plane has a side that is 10 mm in length.

5. A culture system for culturing cell masses, comprising:
a culture tank for housing a culture unit comprising cell masses stacked on a plurality of supports according to any one of claims 1 through 4;
a culture solution container; and
a plurality of culture solution supply pipes for supplying a culture solution from the culture solution container to the culture unit,
wherein each of the plurality of culture solution supply pipes is placed between the needle-shaped parts.

6. The culture system according to claim 5, wherein the culture unit comprises the supports which are arranged adjacent to each other.

7. The culture system according to either one of claims 5 and 6, wherein each of the plurality of culture solution supply pipes is arranged along the top surface of the substrate.

8. The culture system according to any one of claims 5 through 7, wherein each of the plurality of culture solution supply pipes is arranged below the cell masses.

9. The culture system according to any one of claims 5 through 8, wherein each of the plurality of culture solution supply pipes is provided with an opening through which the culture solution can flow out.

10. The culture system according to claim 9, wherein the opening is formed to allow the culture solution to flow out toward the cell masses.

11. The culture system according to any one of claims 5 through 10, wherein each of the plurality of culture solution supply pipes extends over the substrate parallel to at least one side of the substrate.

12. The culture system according to any one of claim 5 through 11, wherein the plurality of culture solution supply pipes are arranged parallel to each other.

13. The culture system according to any one of claims 5 through 12, wherein the culture tank comprises a frame or a hollowed part for housing the culture unit.

14. A method for producing a large-size three-dimensional cellular structure which has a length of at least 10 mm in any one direction, the method comprising placing a culture unit, in which cell masses are stacked on the plurality of supports according to any one of claims 1 through 4, in the culture system according to any one of claims 5 through 13; and conducting culture.

15. The method according to claim 14, wherein the stacking of the cell masses comprises designing shape data of tissue to be produced by dividing the shape data into a plurality of parts and stacking the cell masses on the respective supports based on the divided design data.

16. The method according to either one of claims 14 and 15, wherein the culture unit comprises the stacked cell masses that are positioned to form the original shape of the three-dimensional cellular structure to be produced.

17. A large-size three-dimensional cellular structure produced by the method according to any one of claims 14 through 16 and having a length of at least 10 mm in any one direction.
